# EUROPÄISCHE PATENTANMELDUNG

(11) **EP 0 761 207 A2**
(43) Veröffentlichungstag der Anmeldung: **12.03.1997**
(21) Anmeldenummer: 96108206.2
(22) Anmeldetag: 23.05.1996
(51) Int. Cl.: A61K 9/10

(54) **Pharmazeutische, oral anwendbare Zubereitung enthaltend antaciden Wirkstoffe**

(30) Priorität: 14.08.1995 DE 19529862
(71) Anmelder: RHONE-POULENC RORER GMBH, 50792 Köln (DE)
(72) Erfinder: Jettka, Wilfried, Dr., 50765 Köln (DE); Hager, Jörg-Christian, 50827 Köln (DE); Dürr, Manfred, Dr., 50129 Bergheim-Glessen (DE)
(74) Vertreter: Döring, Wolfgang, Dr.-Ing. Patentanwälte Hauck, Graalfs, Wehnert, Döring, Siemons

(57) **Zusammenfassung**

Es wird eine pharmazeutisch, oral anwendbare Zubereitung beschrieben, wobei die Zubereitung mindestens einen antaciden Wirkstoff sowie weitere Inhaltsstoffe enthält. Die Zubereitung besitzt eine flüssige bis halbfeste Konsistenz, so daß die Zubereitung weitestgehend frei von Konservierungsmitteln ist. Ferner weist die Zubereitung als weitere Inhaltsstoffe mehr als 45 Gew.%, bezogen auf die anwendungsfertige Zubereitung, eines Zuckers und/oder eines Zuckeralkohols und bis zu 40 Gew.%, bezogen auf die anwendungsfertige Zubereitung, eines pharmazeutisch unbedenklichen Lösungsmittels auf.

## Beschreibung

Die vorliegende Erfindung betrifft eine pharmazeutische, oral anwendbare Zubereitung mit den Merkmalen des Oberbegriffs des Patentanspruchs 1.

Oral anwendbare pharmazeutische Zubereitungen mit mindestens einem antaciden Wirkstoff sind seit langem bekannt und im Handel erhältlich. Abhängig von dem jeweiligen Wirkstoff bzw. dem Wirkstoffgemisch werden diese bekannten Antacida als Tabletten, Dragee oder Pulver zur Vorbeugung und/oder zur Behandlung von Magenbeschwerden, insbesondere zur Behandlung bei Übelkeit, Magenkrämpfen, Sodbrennen, Völlegefühl, saurem Aufstoßen, Erbrechen, Blähungen, Magengeschwüren und/oder bei Beschwerden nach Alkohol abusus oder Nikotinmißbrauch, angewendet. Hierfür ist es dann in der Regel erforderlich, daß der Patient die entsprechende Tablette oder das Dragee vor dem Verschlucken gründlich zerkaut, was vielfach zu einem Verkleben von Bestandteilen der Tablette bzw. des Dragees im Zahn- und/oder Gaumenbereich des Mundes führt, so daß derartige Zubereitungen von vielen Patienten als unangenehm empfunden und von daher häufig auch nicht eingenommen werden, obwohl hierfür eine Notwendigkeit besteht.

Um die zuvor beschriebenen Probleme bei der Einnahme der bekannten pharmazeutischen Zubereitungen zu beseitigen, wurde bereits versucht, Suspensionen des Wirkstoffes herzustellen, so daß dann derartige Suspensionen in flüssiger Form dem Patienten dargereicht werden. Hierbei ist es jedoch erforderlich, daß zur Überdeckung des recht unangenehm schmeckenden antaciden Wirkstoffes bzw. Wirkstoffgemisches die bekannten flüssigen Zubereitungen einen relativ hohen Anteil an Aromastoffen aufweisen. Desweiteren bedürfen diese bekannten flüssigen Suspensionen stets eines Konservierungsmittels, wobei die hierfür gebräuchlichen Konservierungsmittel entweder den Geschmack der flüssigen bekannten Zubereitung drastisch verschlechtern, allergische Reaktionen hervorrufen können und/oder im Verdacht stehen, cancerogen zu sein.

Der vorliegenden Erfindung liegt die Aufgabe zugrunde, eine flüssige oder halbfeste pharmazeutische Zubereitung mit einem antaciden Wirkstoff zur Verfügung zu stellen, die einen besonders angenehmen Geschmack aufweist.

Diese Aufgabe wird erfindungsgemäß durch eine pharmazeutische Zubereitung mit den kennzeichnenden Merkmalen des Patentanspruchs 1 gelöst.

Die erfindungsgemäße Zubereitung weist, wie der eingangs aufgeführt Stand der Technik, mindestens einen antaciden Wirkstoff bzw. mindestens ein antacides Wirkstoffgemisch auf und enthält darüber hinaus weitere Inhaltsstoffe. Wesentlich bei der erfindungsgemäßen Zubereitung ist, daß die erfindungsgemäße Zubereitung eine flüssige bis halbfeste Konsistenz besitzt, wobei die erfindungsgemäße Zubereitung weitestgehend auf Konservierungsmittel verzichtet. Darüber hinaus enthält die erfindungsgemäße Zubereitung als weitere Inhaltsstoffe mehr als 45 Gew.%, bezogen auf die anwendungsfertige Zubereitung, eines Zuckers und/oder eines Zuckeralkohols und bis zu 40 Gew.%, bezogen auf die anwendungsfertige Zubereitung, eines pharmazeutisch unbedenklichen Lösungsmittels.

Die erfindungsgemäße Zubereitung weist eine Reihe von Vorteilen auf. Zunächst ist als ein erster Vorteil festzuhalten, daß aufgrund der flüssigen bis halbfesten Konsistenz die erfindungsgemäße Zubereitung besonders einfach einnehmbar ist, da hierbei die zuvor beschriebenen Probleme der bekannten festen Zubereitungen nicht auftreten, d.h. die erfindungsgemäße Zubereitung braucht aufgrund ihrer flüssigen bzw. halbfesten Konsistenz nicht im Mund zerkleinert werden. Aufgrund dieser flüssigen bis halbfesten Konsistenz erlaubt die erfindungsgemäße Zubereitung auch eine individuelle Dosierung, was bei den bekannten Tabletten ebenfalls nicht so ohne weiteres möglich ist. Bedingt dadurch, daß die erfindungsgemäße Zubereitung weitestgehend auf Konservierungsmittel verzichten kann, was bedeutet, daß die erfindungsgemäße Zubereitung in der Regel frei von Konservierungsmitteln ist, treten bei einer derartigen flüssigen bzw. halbfesten erfindungsgemäßen Zubereitung auch keine geschmacklichen Probleme auf, wie dies bei den bekannten Suspensionen der Fall ist. Vielmehr wird bei der erfindungsgemäßen Zubereitung der von dem antaciden Wirkstoff bzw. dem antaciden Wirkstoffgemisch stammende unangenehme Geschmack dadurch kaschiert, daß die erfindungsgemäße Zubereitung mindestens 45 Gew.% eines Zuckers und/oder eines Zuckeralkohols enthält, so daß dieser Zucker bzw. Zuckeralkohol in der erfindungsgemäßen Zubereitung nicht nur den unangenehmen Wirkstoffgeschmack kaschiert sondern auch dann der erfindungsgemäßen Zubereitung einen angenehmen Geschmack verleiht, wenn diese geringen Mengen eines Konservierungsmittels, beispielsweise zwischen 0,01 Gew.% und etwa 0,1 Gew.%, enthält. Überraschend konnte festgestellt werden, daß die erfindungsgemäße Zubereitung auch ohne jegliches Konservierungsmittels mikrobiologisch stabil und somit autosteril ist, wobei diese positive Eigenschaft der erfindungsgemäßen Zubereitung auf die zuvor genannte hohe Konzentration des Zuckers bzw. des Zuckeralkohols in der erfindungsgemäßen Zubereitung zurückgeführt wird. Trotz des relativ hohen Anteils an Zuckers bzw. Zuckeralkohol in der erfindungsgemäßen Zubereitung neigt die erfindungsgemäße Zubereitung nicht zu einer Sedimentation oder zu einem Ausklumpen des antaciden Wirkstoffes bzw. Wirkstoffgemisches, so daß die flüssige bis halbfeste erfindungsgemäße Zubereitung in sich stabil ist, so daß es nicht erforderlich wird, die erfindungsgemäße Zubereitung vor der Einnahme zu schütteln, um so eine Vergleichmäßigung des Wirkstoffes bzw. des Wirkstoffgemisches in der erfindungsgemäßen Zubereitung herbeizuführen. Weiterhin bewirkt die flüssige bis halbfeste Konsistenz der erfindungsgemäßen Zubereitung, daß sich der antacide Wirkstoff bzw. das antacide Wirkstoffgemisch nach Einnahme der erfindungsgemäßen Zubereitung sehr schnell und gleichmäßig im Magen und Darmtrakt des Patienten verteilen kann, so daß sich mit der erfindungsgemäßen Zubereitung eine schnelle Linderung der Beschwerden und rasche Heilung herbeiführen läßt. Auch läßt sich die erfindungsgemäße Zubereitung aufgrund ihrer flüssigen bis halbfesten Formulierung sehr genau und reproduzierbar entnehmen oder in Einzeldosen abpacken, so daß dementsprechend der Herstellungsprozeß auch standardisiert ist.

Eine erste Ausführungsform der erfindungsgemäßen Zubereitung sieht vor, daß der Feststoffgehalt der flüssigen bis halbfesten Zubereitung bei maximal 88 Gew.%, bezogen auf die anwendungsfertige Zubereitung, liegt. Hierbei enthält eine derartige Ausführungsform der erfindungsgemäßen Zubereitung als Feststoffe mindestens den einen antaciden Wirkstoff bzw. das Wirkstoffgemisch sowie den Zucker und/oder den Zuckeralkohol in der vorstehend genannten Konzentration, wobei mindestens noch 12 Gew.%, bezogen auf die anwendungsfertige Zubereitung, des zuvor genannten pharmazeutisch unbedenklichen Lösungsmittels als Suspensions- bzw. Emulgiermittel in der flüssigen bis halbfesten erfindungsgemäßen Zubereitung enthalten sind. Überraschend konnte bei einer derartigen, an Feststoffgehalt reichen Ausführungsform der erfindungsgemäßen Zubereitung festgestellt werden, daß es bei dieser Ausführungsform selbst dann noch nicht zu einem Verklumpen und/oder einer Sedimentation des antaciden Wirkstoffes kommt, wenn der Feststoffgehalt zwischen 65 Gew.% und 88 Gew.%, bezogen auf die anwendungsfertige Zubereitung, variiert. Auch nach einer Lagerzeit von mehreren Monaten wies eine derartige Ausführungsform eine homogene Zusammensetzung und gleichmäßige Verteilung des antaciden Wirkstoffes bzw. Wirkstoffgemisches auf.

Bezüglich des in der erfindungsgemäßen Zubereitung enthaltenen Lösungsmittels ist festzuhalten, daß es sich hierbei insbesondere um ein pharmazeutisch unbedenkliches Lösungsmittel handelt. Vorzugsweise weist die erfindungsgemäße Zubereitung als Lösungsmittel Wasser, Ethanol, Propanol-1 und/oder Propanol-2 jeweils allein oder in Mischung auf, wobei von den zuvor aufgeführten Lösungsmitteln dann insbesondere Wasser wegen seiner absoluten Unbedenklichkeit bevorzugt wird. Unter dem Begriff Wasser werden im Rahmen der vorliegenden Anmeldung alle wäßrigen Systeme subsumiert.

Eine besonders geeignete und hochwirksame erfindungsgemäße Zubereitung weist als pharmazeutisch unbedenkliches Lösungsmittel zwischen 12 Gew.% und 35 Gew.% Wasser, bezogen auf die anwendungsfertige Zubereitung, auf. Hier konnte festgestellt werden, daß sich diese Ausführungsform der erfindungsgemäßen Zubereitung äußerst angenehm einnehmen läßt, da diese Ausführungsform besonders geschmacksneutral ist, zumal der unangenehme Eigengeschmack des antaciden Wirkstoffes bzw. Wirkstoffgemisches vollständig durch die hohe Konzentration an Zucker und/oder Zuckeralkohol in der Zubereitung kaschiert wird. Auch konnten in einer derartigen wäßrigen, flüssigen bis halbfesten Formulierung der erfindungsgemäßen Zubereitung keine Ausflockungen des Wirkstoffes bzw. Wirkstoffgemisches oder eine Phasentrennung der Zubereitung festgestellt werden, selbst dann nicht, wenn die erfindungsgemäße Zubereitung längere Zeit ohne Schütteln gelagert wird.

Auch bezüglich der Konzentration des antaciden Wirkstoffes bzw. des antaciden Wirkstoffgemisches in der erfindungsgemäßen Zubereitung ist festzuhalten, daß sich diese Konzentration danach richtet, welche Tagesdosen der erfindungsgemäßen Zubereitung gegeben werden. Insbesondere weist die erfindungsgemäße Zubereitung zwischen 5 Gew.% und 43 Gew.%, vorzugsweise zwischen 12 Gew.% und 30 Gew.%, des antaciden Wirkstoffes auf, wobei diese zuvor angegebenen Konzentrationen jeweils auf die anwendungsfertige Zubereitung bezogen sind.

Bezüglich des in der erfindungsgemäßen Zubereitung enthaltenen mindestens einen antaciden Wirkstoffes bzw. des antaciden Wirkstoffgemisches ist festzuhalten, daß es sich hierbei die an sich bekannten antaciden Wirkstoffe handelt, vorzugsweise um Aluminiumhydroxid, Magnesiumhydroxid, Magnesiumtrisilikat, Magnesiumcarbonat, Magnesiumphosphat, Calciumcarbonat, Calciumphosphat, Natriumcitrat, Magnesiumoxid, Magaldrat, Hydrotalcit, Natriumhydrogencarbonat und/oder Wismutsubcarbonat, wobei die zuvor genannten Wirkstoffkonzentration sich auf die wasserfreie Wirkstoffsubstanz beziehen.

Wie bereits vorstehend ausgeführt ist, weist die erfindungsgemäße Zubereitung als weiteren Inhaltsstoff mehr als 45 Gew.%, bezogen auf die anwendungsfertige Zubereitung, des Zuckers und/oder des Zuckeralkohols auf. Insbesondere variiert in der erfindungsgemäßen Zubereitung die Konzentration des Zuckers und/oder des Zuckeralkohols zwischen 45 Gew.% und 80 Gew.%, vorzugsweise zwischen 60 Gew.% und 80 Gew.%. Trotz dieser relativ hohen Konzentration an Zucker und/oder Zuckeralkohol in der erfindungsgemäßen Zubereitung weist die flüssige bis halbfeste Formulierung der erfindungsgemäßen Zubereitung noch eine ausgezeichnete Stabilität auf, so daß es weder bei der Herstellung noch bei einer extrem langen Lagerzeit zu einem Ausflocken, Ausklumpen oder Ausfällen des antaciden Wirkstoffes bzw. antaciden Wirkstoffgemisches kommt. Darüber hinaus besitzen die zuvor genannten Ausführungsformen der erfindungsgemäßen Zubereitung, die einen Zuckergehalt bzw. eine Konzentration an Zuokeralkohol zwischen 45 Gew.% und 80 Gew.% aufweisen, einen äußerst angenehmen Geschmack auf und sind zudem noch autosteril, so daß sie sehr gern von den Patienten, insbesondere auch von jugendlichen Patienten, eingenommen werden.

Unter den Begriff Zucker im Sinne der vorliegenden Anmeldung fallen alle an sich bekannten und in der pharmazeutischen Industrie üblicherweise eingesetzten Mono-, Di- und/oder Oligosaccharide, vorzugsweise jedoch Saccharose, Glukose, Fructose, Maltose, Lactose, Galaktose und/oder Stärkehydrolysate. Vorzugsweise sieht eine Ausführungsform der erfindungsgemäßen Zubereitung vor, daß diese als Zucker Saccharose enthält.

Unter den Begriff Zuckeralkohole im Sinne der vorliegenden Anmeldung fallen alle an sich bekannten und in der pharmazeutischen Industrie üblicherweise eingesetzten mono-, di- und oligomeren hydrierten Zucker, vorzugsweise jedoch Sorbitol, Mannitol, Xylitol, Malitol und/oder hydrierte Stärkehydrolysate.

Eine besonders geeignete Ausführungsform der erfindungsgemäßen Zubereitung weist als Zuckeralkohol Sorbitol, Xylitol und/oder Maltitol in den zuvor genannten Konzentrationen auf. Hierbei zeichnet sich diese Ausführungsform insbesondere durch einen äußerst angenehmen Geschmack und einer hohen mikrobiologischen Stabilität aus, so daß diese Ausführungsform der erfindungsgemäßen Zubereitung besonders gern und unproblematisch genommen wird.

Um die Einnahme der erfindungsgemäßen Zubereitung weiter zu erleichtern, sieht eine besonders vorteilhafte Weiterbildung der erfindungsgemäßen Zubereitung vor, daß hierbei die erfindungsgemäße Zubereitung eine gelartige Konsistenz besitzt. Insbesondere dann, wenn eine derartige gelartige Zubereitung in Einzeldosen verpackt wird, beispielsweise in entsprechenden Beuteln abgepackt und verschweißt wird, kann der Patient im Bedarfsfall sehr schnell und einfach eine dosierbare Menge der erfindungsgemäßen Zubereitung zu sich nehmen, ohne daß dabei die Gefahr besteht, daß ein Teil der erfindungsgemäßen Zubereitung verlorengeht.

Bezüglich der weiteren Inhaltsstoffe der erfindungsgemäßen Zubereitung ist festzuhalten, daß es sich hierbei um solche pharmazeutisch üblichen Inhaltsstoffe handelt, die üblicherweise in einer flüssigen bis halbfesten Zubereitung enthalten sind. Um z.B. bei der erfindungsgemäßen Zubereitung eine gewünschte Konsistenz, beispielsweise die zuvor aufgeführte gelartige Konsistenz, sicherzustellen, kann die erfindungsgemäße Zubereitung mindestens ein geeignetes Verdickungsmittel, insbesondere zwischen 0,2 Gew.% und 2 Gew.%, enthalten, wobei sich hierfür insbesondere Xanthan Gum, Guar Gum und/oder Cellulosederivate, vorzugsweise mikrokristalline Cellulose, Methylcellulose, Hydroxyethylcellulose, Methylhydroxypropylcellulose und/oder Hydroxypropylcellulose, als besonders geeignet erwiesen haben. Ferner bietet es sich an, daß die erfindungsgemäße Zubereitung desweiteren als Inhaltsstoffe zwischen 2 Gew.% und 8 Gew.% Glycerol sowie zwischen 0,05 Gew.% und 0,7 Gew.% eines Aromastoffes aufweist.

Eine besonders bevorzugte Formulierungen der erfindungsgemäßen Zubereitung weist als Hauptbestandteile
- 12 Gew.% - 28 Gew.%: des antaciden Wirkstoffes bzw. Wirkstoffgemisches,
- 46 Gew.% - 58 Gew.%: des Zuckers und/oder Zuckeralkohols, sowie
- 18 Gew.% - 30 Gew.%: Wasser
auf, wobei in dieser Zubereitung dann ggf. 0 - 10 Gew.% weitere Bestandteile, insbesondere Glycerol, Verdickungsmittel und/der Aromastoffe, enthalten sind.

Vorteilhafte Weiterbildungen der erfindungsgemäßen Zubereitung sind in den Unteransprüchen angegeben.

Die erfindungsgemäße Zubereitung wird nachfolgend anhand von drei Ausführungsbeispielen näher erläutert.

### Ausführungsbeispiel 1

Es wurde eine gelartige Formulierung hergestellt, die die nachfolgenden Inhaltsstoffe aufwies:
- 13,33 g: Aluminiumoxid, wasserhaltig, mit einer Konzentration von 50 Gew.% Al₂O₃ (trocken);
- 13,33 g: Magnesiumhydroxidpulver, wasserfrei;
- 52,5 g: Sorbitol-Lösung, 70 Gew.% in Wasser, nicht kristallisierend;
- 15,0 g: Maltitol-Lösung, 80 Gew.% in Wasser (Lycasin);
- 5,34 g: Glycerol, 85 Gew.%,
- 0,35 g: Xanthan Gum; und
- 0,15 g: Aromastoffe.

Zur Herstellung dieser gelartigen Formulierung wurde die Lösungen der Zuckeralkohole zunächst mit dem Glycerol versetzt. Anschließend wurde in diese Mischung das wasserhaltige Aluminiumoxid sowie die Magnesiumhydroxid suspendiert. Die so hergestellte Mischung wurde nach hinreichendem Rühren mit den Aromastoffen versetzt und anschließend mit dem Xanthan Gum verdickt.

Die so hergestellte gelartige Formulierung wies eine Konzentration an antaciden Wirkstoff von 26,7 Gew.%, einen Wassergehalt von 19,6 Gew.% sowie einen Gehalt an Zuckeralkoholen von 48,8 Gew.% auf, wobei diese Angaben alle auf die anwendungsfertige Zubereitung bezogen sind.

### Ausführungsbeispiel 2

Es wurde eine zweite gelartige Formulierung hergestellt, die die folgenden Hauptbestandteile aufwies:
- 10,00 g: Aluminiumoxid, wasserhaltig, mit einem Feststoffgehalt von Al₂O₃ von 50 Gew.%;
- 10,00 g: Magnesiumhydroxidpulver, wasserfrei;
- 79,86 g: Zucker-Sirup (64 Gew.% Trockensubstanz) ; und
- 0,14 g: Aromastoffe.

Zur Herstellung dieser Formulierung wurde der wäßrige Zucker-Sirup vorgelegt. In den wäßrigen Zucker-Sirup wurden das wasserhaltige Aluminiumoxid und das Magnesiumhydroxid-Pulver suspendiert. Anschließend erfolgte ein Zusatz der Aromastoffe. Nach hinreichendem Rühren entstand eine gelartige Formulierung, die eine Konzentration an antacidem Wirkstoffgemisch von 20 Gew.% (berechnet als wasserfreie Substanz), einen Wassergehalt von 28,7 Gew.% und einen Gehalt an Zucker von 51,1 Gew.% (berechnet als wasserfreie Substanz), jeweils bezogen auf die anwendungsfertige Zubereitung, enthielt.

### Ausführungsbeispiel 3

Es wurde eine dritte halbfeste Formulierung erstellt, wobei diese halbfeste Formulierung folgende Inhaltsstoffe aufwies:
- 13,3 g: Calciumcarbonat;
- 62,0 g: Sorbitol-Lösung in Wasser, 70 Gew.%, nicht kristallisierend;
- 17,8 g: Maltitol-Lösung in Wasser, 80 Gew.% (Lycasin);
- 6,31 g: Glycerol, 85 Gew.%ig;
- 0,42 g: Xanthan Gum; und
- 0,17 g: Aromastoffe.

Die Herstellung der Formulierung gemäß Ausführungsbeispiel 3 erfolgte genau so, wie dies vorstehend für die Formulierung gemäß Ausführungsbeispiel 1 beschrieben ist.

Die anwendungsfertige, halbfeste Formulierung 3 wies eine Konzentration von 13,3 Gew.% des antaciden Wirkstoffes (berechnet als wasserfreie Substanz), 22,3 Gew.% Wasser sowie 57,6 Gew.% des Zuckeralkohols (berechnet als wasserfreie Substanz) auf.

Zur Überprüfung der Akzeptanz wurde die gelartige Formulierung gemäß Ausführungsbeispiel 1, die die höchste Konzentration an bitterschmeckenden antaciden Wirkstoffen aufwies, an 50 freiwillige Testpersonen verteilt, wobei bei dieser orientierenden Geschmacksuntersuchung als Vergleich eine herkömmliche, auf dem Markt befindliche Suspension herangezogen wurde.

Zur Objektivierung dieses Geschmacksversuches erhielten die 50 Testpersonen zunächst dreimal die Formulierung gemäß Ausführungsbeispiel 1, wobei zwischen jedem Test eine Woche Zeit lag.

Nach einer geschmacklichen Neutralisier-Phase, d.h. einer Pause von drei Stunden mit wiederholtem Trinken von Wasser, wurde dann die herkömmliche Suspension gereicht.

In allen drei Tests berichteten 47 Testpersonen, daß die Formulierung gemäß Ausführungsbeispiel 1 einen wesentlich angenehmeren Geschmack aufwies als die herkömmliche Suspension, wobei 44 Testpersonen berichteten, daß die Formulierung gemäß Ausführungsbeispiel 1 bereits wenige Minuten nach dem Probieren auch keinen süßen Nachgeschmack mehr hervorgerufen hatte.

Der vorstehend beschriebene 3-Phasen-Test wurde nach einem Monat wiederholt, wobei die selben Testpersonen hierbei zunächst die herkömmliche Suspension probierten und nach einer dreistündigen Pause mit wiederholtem Trinken von Wasser die Formulierung gemäß Ausführungsbeispiel 1 kosteten.

Hierbei berichteten übereinstimmend 49 Personen, daß die Formulierung gemäß Ausführungsbeispiel 1 wesentlich angenehmer im Geschmack war, während die herkömmliche Suspension selbst trotz mehrfachen Spülen mit Wasser noch einen unangenehmen Nachgeschmack besaß.

Zum Nachweis der mikrobiologischen Stabilität der Formulierung gemäß Ausführungsbeispiel 1 wurde folgende Untersuchung durchgeführt:

Es wurde eine Prüfung der Formulierung gemäß Ausführungsbeispiel 1 auf antimikrobielle Konservierung nach DAB 10, 3. Nachtrag 1994, durchgeführt.

Das Untersuchungsergebnis dieser Überprüfung ist in der nachfolgenden Tabelle 1 wiedergegeben, wobei die Beimpfung mit 0,3 ml Keimsuspension erfolgte.

**Tabelle 1**

| Teststamm Lagerzeit | Einsaat | Keimzahl pro g/ml nach einer | | | |
|---|---|---|---|---|---|
| | | von sofort | 14 Tage | Red.F. | 28 |
| Tage | | | | | |
| 1 | 4.909.000 | 4.800.000 | 240 | 10⁴ | 230 |
| 2 | 4.991.000 | 800.000 | 230 | 10⁴ | 260 |
| 3 | 3.569.000 | 275.680 | 200 | 10⁴ | 250 |
| 4 | 3.169.000 | 36.036 | 220 | 10⁴ | 250 |
| 5 | 1.500.000 | 900.000 | 180 | > 10³ | 100 |
| 6 | 3.918.000 | 1.703.000 | 200 | | 164 |
| 7 | 1.455.000 | 53.423 | 250 | | 800 |
| 8 | | 250 | 200 | | 170 |
| 1 = Staph. aureus 2 = Escherichia coli 3 = Ps. aeruginosa 4 = Candida albicans 5 = Aspergillus niger 6 = Zygosacch. rouxii 7 = Schmutzwasser 8 = Kontrolle, unbeimpft | | | | | |

## Patentansprüche

1. Pharmazeutische, oral anwendbare Zubereitung, wobei die Zubereitung mindestens einen antaciden Wirkstoff sowie weitere Inhaltsstoffe enthält, dadurch gekennzeichnet, daß die Zubereitung eine flüssige bis halbfeste Konsistenz besitzt, daß die Zubereitung weitestgehend frei von Konservierungsmitteln ist und daß die Zubereitung als weitere Inhaltsstoffe mehr als 45 Gew.%, bezogen auf die anwendungsfertige Zubereitung, eines Zuckers und/oder eines Zuckeralkohols und bis zu 40 Gew.%, bezogen auf die anwendungsfertige Zubereitung, eines pharmazeutisch unbedenklichen Lösungsmittels aufweist.

2. Pharmazeutische Zubereitung nach Anspruch 1, dadurch gekennzeichnet, daß der Feststoffgehalt der flüssigen bis halbfesten Zubereitung bei maximal 88 Gew.%, bezogen auf die anwendungsfertige Zubereitung, liegt.

3. Pharmazeutische Zubereitung nach Anspruch 1 oder 2, dadurch gekennzeichnet, daß die Zubereitung als pharmazeutisch unbedenkliches Lösungsmittel Wasser enthält.

4. Pharmazeutische Zubereitung nach einem der vorangehenden Ansprüche, dadurch gekennzeichnet, daß die Zubereitung zwischen 12 Gew.% und 35 Gew.% Wasser, bezogen auf die anwendungsfertige Zubereitung, aufweist.

5. Pharmazeutische Zubereitung nach einem der vorangehenden Ansprüche, dadurch gekennzeichnet, daß die Zubereitung zwischen 5 Gew.% und 43 Gew.%, vorzugsweise zwischen 12 Gew.% und 30 Gew.%, des antaciden Wirkstoffes, bezogen auf die anwendungsfertige Zubereitung, aufweist.

6. Pharmazeutische Zubereitung nach einem der vorangehenden Ansprüche, dadurch gekennzeichnet, daß die Zubereitung als antaciden Wirkstoff Aluminiumhydroxid, Magnesiumhydroxid, Magnesiumtrisilikat, Magnesiumcarbonat, Magnesiumphosphat, Calciumcarbonat, Calciumphosphat, Natriumcitrat, Magnesiumoxid, Magaldrat, Hydrotalcit, Natriumhydrogencarbonat und/oder Wismutsubcarbonat enthält.

7. Pharmazeutische Zubereitung nach einem der vorangehenden Ansprüche, dadurch gekennzeichnet, daß die Zubereitung den Zucker und/oder den Zuckeralkohol in einer Konzentration zwischen 45 Gew.% und 80 Gew.%, vorzugsweise zwischen 48 Gew.% und 70 Gew.%, jeweils bezogen auf die anwendungsfertige Zubereitung, enthält.

8. Pharmazeutische Zubereitung nach einem der vorangehenden Ansprüche, dadurch gekennzeichnet, daß die Zubereitung als Zuckeralkohol Sorbitol und/oder Maltitol enthält.

9. Pharmazeutische Zubereitung nach einem der vorangehenden Ansprüche, dadurch gekennzeichnet, daß die Zubereitung als Zucker Saccharose enthält.

10. Pharmazeutische Zubereitung nach einem der vorangehenden Ansprüche, dadurch gekennzeichnet, daß die Zubereitung eine gelartige Konsistenz besitzt.

11. Pharmazeutische Zubereitung nach einem dem vorangehenden Ansprüche, dadurch gekennzeichnet, daß die Zubereitung desweiteren als Inhaltsstoffe zwischen 2 Gew.% und 8 Gew.% Glycerol, zwischen 0,2 Gew.% und 2 Gew.% eines Verdickungsmittels, insbesondere Xanthan Gum, Guar Gum und/oder ein Cellulosederivat, vorzugsweise mikrokristalline Cellulose, Methylcellulose, Hydroxyethylcellulose, Methylhydroxypropylcellulose und/oder Hydroxypropylcellulose, und zwischen 0,05 Gew.% und 0,7 Gew.% eines Aromastoffes aufweist.

12. Pharmazeutische Zubereitung nach einem der vorangehenden Ansprüche, dadurch gekennzeichnet, daß die Zubereitung als Hauptbestandteile
12 Gew.% - 28 Gew.% des antaciden Wirkstoffes bzw. Wirkstoffgemisches,
46 Gew.% - 58 Gew.% des Zuckers und/oder Zuckeralkohols,
18 Gew.% - 30 Gew.% Wasser
aufweist.
